(19) Europäisches Patentamt
European Patent Office
Office européen des brevets

(11) EP 0 657 410 B1

(12) **EUROPÄISCHE PATENTSCHRIFT**

(45) Veröffentlichungstag und Bekanntmachung des Hinweises auf die Patenterteilung:
**19.11.1997 Patentblatt 1997/47**

(51) Int. Cl.[6]: **C07C 43/11**, C07C 41/03

(21) Anmeldenummer: **94118951.6**

(22) Anmeldetag: **01.12.1994**

(54) **Verfahren zur Herstellung von Alkoxylaten unter Verwendung von Esterverbindungen als Katalysator**

Process for the preparation of alkoxylates using ester compounds as catalysts

Procédé pour la préparation d'alkoxylates utilisant des esters comme catalyseurs

(84) Benannte Vertragsstaaten:
**AT BE CH DE ES FR GB IT LI NL SE**

(30) Priorität: **07.12.1993 DE 4341576**

(43) Veröffentlichungstag der Anmeldung:
**14.06.1995 Patentblatt 1995/24**

(73) Patentinhaber: **Clariant GmbH**
**65929 Frankfurt am Main (DE)**

(72) Erfinder:
- **Wehle, Detlef, Dr.**
  **D-65611 Niederbrechen (DE)**
- **Kremer, Gernot, Dr.**
  **D-65779 Kelkheim (DE)**
- **Wimmer, Ignaz**
  **D-84543 Winhöring (DE)**

(56) Entgegenhaltungen:
**EP-A- 0 295 578**
**EP-A- 0 337 239**
**EP-A- 0 566 956**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.14.23/3.4

EP 0 657 410 B1

## Beschreibung

Alkoxylate haben große Bedeutung, zum Beispiel als Zwischenprodukte für Derivatisierungen und als nichtionogene Komponenten in technischen und kosmetischen Wasch- und Reinigungsmitteln. Sie werden ferner in einer Vielzahl von Anwendungsgebieten als Emulgatoren, Dispergatoren und dergleichen eingesetzt. Dabei werden häufig solche Alkoxylate gewünscht, die eine enge Verteilung der Alkoxylierungshomologen aufweisen. Diese Verteilung wird im wesentlichen durch den bei der Alkoxylierungsreaktion verwendeten Katalysator bestimmt.

Es ist schon seit langem bekannt, zum Beispiel aus der britischen Patentschrift 796 508, daß Antimonpentahalogenide als Katalysator bei der Umsetzung von aktive H-Atome (zum Beispiel in Form von Hydroxylgruppen) enthaltenden Verbindungen mit Alkylenoxid (zum Beispiel Ethylenoxid und/oder Propylenoxid) zu Alkoxylaten mit enger Homologenverteilung führen ("narrow-range"-Alkoxylate). Diesem Vorteil steht als Nachteil die schwierige Handhabbarkeit der Antimonpentahalogenide (stark rauchend, korrosiv, hydrolyseempfindlioh) und die nicht zufriedenstellende Farbqualität des Alkoxylates entgegen.

In der US-Patentschrift 4 996 364 werden unsubstituierte Polycarbonsäuremonoester in Form von Erdalkalimetallsalzen als Katalysator für die Alkoxylierung von Verbindungen mit aktiven H-Atomen wie Fettalkoholen beschrieben. Als Polycarbonsäure wird unter anderen auch die Bernsteinsäure genannt, jedoch wiederum unsubstituiert. Diese Erdalkalisalze von Polycarbonsäuremonoestern weisen im Vergleich zu Antimonpentahalogeniden als Katalysator zwar Vorteile auf, sie lassen aber hinsichtlich Homologenverteilung und/oder Aussehen der erhaltenen Alkoxylate noch immer zu wünschen übrig.

In der canadischen Patentanmeldung 2 094 556 werden Alkalimetall- oder Erdalkalimetallsalze von Alkyl- oder Alkenylbernsteinsäuremonoestern als Korrosionsschutzmittel für die Metallbearbeitung beschrieben, wobei der Alkyl- oder Alkenylsubstituent 8 bis 30 C-Atome enthält, vorzugsweise 9 bis 15 C-Atome. Ein Hinweis oder eine Anregung in Richtung auf irgendeine andere Vervendungsmöglichkeit dieser Halbester der substituierten Bernsteinsaure wird in der Druckschrift nicht gegeben.

Das erfindungsgemäße Verfahren zur Herstellung von Alkoxylaten durch Alkoxylierung von mindestens ein aktives H-Atom enthaltenden Verbindungen in Gegenwart eines Salzes von einem Bernsteinsäuremonoester als Katalysator ist dadurch gekennzeichnet, daß man in Gegenwart von mindestens einem Erdalkalimetallsalz der Alkyl- oder Alkenylbernsteinsäuremonoester der nachstehenden Formeln (I) und (II) alkoxyliert

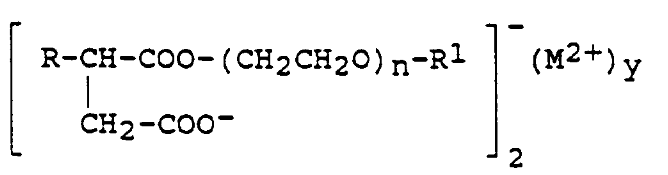 (I)

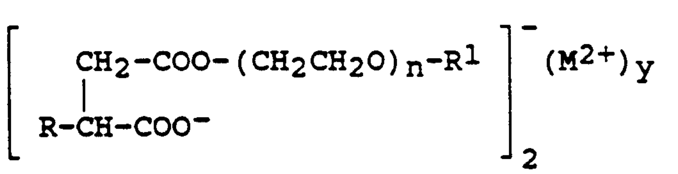 (II)

worin bedeuten

- R $C_8$ bis $C_{30}$-Alkyl oder $C_8$ bis $C_{30}$-Alkenyl,
- n eine Zahl von 0 bis 6,
- $R^1$ $C_1$ bis $C_{18}$-Alkyl oder $C_3$ bis $C_{18}$-Alkenyl oder auch Wasserstoff, wenn n 1 oder > 1 ist,
- M Ba, Ca oder Sr und
- y eine Zahl von 0,9 bis 1,8,

wobei 10 bis 70 % der titrierbaren Alkalität des Bernsteinsäuremonoestersalzes der Formeln (I) und (II) mit einer anorganischen Säure, die mit den Kationen Ba, Ca und Sr in Wasser schwerlösliche Salze bildet, neutralisiert worden sind.

R in den Formeln (I) und (II) ist vorzugsweise ein $C_9$ bis $C_{20}$-Alkyl oder ein $C_9$ bis $C_{20}$-Alkenyl, n ist vorzugsweise eine Zahl von 0 bis 3, $R^1$ ist vorzugsweise ein $C_1$ bis $C_{12}$-Alkyl oder ein $C_3$ bis $C_{12}$-Alkenyl und kann vorzugsweise auch

das Wasserstoffatom sein, wenn n 1 oder > 1 ist, und y ist vorzugsweise eine Zahl von 1 bis 1,3 und besonders bevorzugt 1. M ist aus Zweckmäßigkeitsgründen vorzugsweise Ca. Die Alkenylgruppen haben vorzugsweise 1 bis 3 Doppelbindungen. Die Alkyl- und Alkenylgruppen können gerade oder verzweigt sein. Alkyl und Alkenyl können auch in Form von Gemischen vorliegen, zum Beispiel in Form eines Gemisches aus $C_{12}$ und $C_{14}$-Alkyl ($C_{12/14}$-Alkyl) oder $C_{12}$ und $C_{14}$-Alkenyl ($C_{12/14}$-Alkenyl) oder aus $C_{12}$ und $C_{14}$-Alkyl- und -Alkenylgruppen. Von den zu verwendenden Erdalkalisalzen von einem mit einer Alkylgruppe oder einer Alkenylgruppe substituierten Bernsteinsäurehalbester sind jene mit einer Alkenylgruppe substituierten bevorzugt, das heißt, R in den Formeln (I) und (II) ist vorzugsweise eine der genannten Alkenylgruppen. Der vom Veresterungsalkohol resultierende Rest $R^1$ ist dagegen vorzugsweise eine der genannten Alkylgruppen. Beispiele für Alkyl- und Alkenylreste sind also Methyl, Propyl, Butyl, Iso-butyl, Octyl, Octenyl, Decyl, Decenyl, Dodecyl (Lauryl), Dodecenyl, Oleyl, Octadecadienyl, Octadecatrienyl und Talgfettalkyl.

Es ist bevorzugt, in Gegenwart von mindestens einem Bernsteinsäuremonoestersalz der Formeln (I) und (II) zu alkoxylieren, von dem 30 bis 60 % seiner titrierbaren Alkalität neutralisiert worden sind mit einer anorganischen Säure, die mit den Kationen Ba, Ca und Sr in Wasser schwerlösliche Salze bildet. Bevorzugte Mineralsäuren sind Schwefelsäure ($H_2SO_4$) und schwefelige Säure ($H_2SO_3$) sowie Phosphorsäure ($H_3PO_4$) und phosphorige Säure ($H_3PO_3$).

Die zu verwendenden Erdalkalisalze von Alkyl- und/oder Alkenylbernsteinsäurehalbestern (das sind Stellungsisomere, die im allgemeinen in Mischung vorliegen) sind bekannt und im Handel erhältlich, zum Beispiel als ®HOSTACOR (® = eingetragenes Warenzeichen von Hoechst). Sie werden bevorzugt in der Weise hergestellt, daß man entsprechende Alkyl- oder Alkenylbernsteinsäureanhydride oder auch die entsprechenden Säureverbindungen mit Alkoholen der Formel $R^1$-OH oder alkoxylierten Alkoholen der Formel $R^1$-$(OCH_2CH_2)_n$-OH, wobei $R^1$ und n die genannten Bedeutungen haben, in äquimolaren Mengen umsetzt (verestert) und aus den erhaltenen Alkyl- oder Alkenylbernsteinsäurehalbestern mit basischen Erdalkaliverbindungen, das sind zum Beispiel die Acetate, Oxide, Carbonate oder Hydroxide, die entsprechenden Erdalkalisalze herstellt. Die Veresterung wird vorzugsweise bei einer Temperatur von 60 bis 150 °C ohne Verwendung eines Lösungsmittels unter Stickstoffatmosphäre durchgeführt. Der Halbester wird dann durch Umsetzung mit vorzugsweise einer Oxid-, Carbonat- oder Hydroxidverbindung des Bariums, Calciums oder Strontiums, wobei die Hydroxide bevorzugt sind, in das Salz dieser Erdalkalimetalle übergeführt. Die Erdalkalimetallverbindung wird entsprechend dem Wert y in den Formeln (I) und (II) in einer Molmenge von 0,9 bis 1,8 mol, vorzugsweise 1 bis 1,3 mol und besonders bevorzugt in einer Menge von 1 mol, pro 2 mol Halbesterverbindung eingesetzt. Die Umsetzung von Halbester und Erdalkalimetallverbindung wird vorzugsweise bei einer Temperatur von 60 bis 160 °C und unter Verwendung eines niedrigsiedenden inerten organischen Lösungsmittels, wie Toluol und Xylol, und/oder eines höhersiedenden inerten organischen Lösungsmittels, wie Kohlenwasserstoffe, Acetale und Ether mit einem Siedepunkt > 150 °C bei Normaldruck, durchgeführt. Das entstehende Reaktionswasser wird ausgetragen. Die Reaktionszeit beträgt etwa 2 Stunden. Nach beendeter Salzbildung wird das niedrigsiedende Lösungsmittel zum Beispiel am Rotationsverdampfer entfernt. Man erhält so im allgemeinen eine etwa 60gew.%ige Lösung der einzusetzenden Bernsteinsäurehalbestersalze in Form eines Öles. Dies setzt voraus, daß als Lösungsmittel ein Gemisch aus niedrig- und höhersiedenden Lösungsmitteln eingesetzt worden ist.

Die Teilneutralisation der titrierbaren Alkalität der Erdalkalisalze der Alkyl- oder Alkenylbernsteinsäurehalbester wird bevorzugt in der Weise durchgeführt, daß man die Mineralsäure in Form einer 10 bis 50gew.%igen wäßrigen Lösung zum Bernsteinsäurehalbester (bei etwa 20 bis 100 °C) in der stöchiometrischen Menge dazugibt, die zur angestrebten Teilneutralisation (der vorher bestimmten titrierbaren Alkalität) erforderlich ist, worauf das im Reaktionsprodukt anwesende Wasser durch Erwärmen auf etwa 50 bis 150 °C, gegebenenfalls unter Anwendung von Vakuum, entfernt wird. Man erhält ein Produkt mit flüssiger bis halbfester Konsistenz.

Die Menge an erfindungsgemäßem Katalysator kann in weiten Grenzen variieren und liegt im allgemeinen bei 0,1 bis 5 Gew.-%, vorzugsweise 0,5 bis 3 Gew.-%, Gewichtsprozente bezogen auf das Gewicht des zu alkoxylierenden Produktes. Der Katalysator wird dem zu alkoxylierenden Produkt in der angegebenen Menge zugesetzt. Er kann auch in situ erzeugt werden, zum Beispiel in der Weise, daß man zunächst dem zu alkoxylierenden Fettalkohol eine definierte Menge von einem Barium-, Calcium- oder Strontiumoxid, -carbonat oder -hydroxid, wobei die Hydroxide bevorzugt sind, und die darauf abgestimmte, aus den Formeln (I) und (II) resultierende stöchiometrische Menge an substituierter Bernsteinsäureanhydridverbindung zusetzt und dann trocknet, gegebenenfalls unter Anwendung von Vakuum, worauf mit der Alkoxylierung begonnen wird. Die in-situ-Variante kann auch die beschriebene Teilneutralisation umfassen.

Die Alkoxylierung der Verbindungen mit aktiven H-Atomen mit Hilfe der erfindungsgemäßen Produkte als Katalysator wird in üblicher Weise durchgeführt, das heißt bei einer Temperatur von 60 bis 200 °C, vorzugsweise 100 bis 180 °C, und einem Druck von etwa 0,5 bis 6 bar, wobei das Alkylenoxid portionsweise oder kontinuierlich zudosiert wird. Die Menge an Alkylenoxid liegt im allgemeinen bei 1 bis 30 mol, vorzugsweise 2 bis 20 mol und insbesondere 2 bis 15 mol pro mol zu alkoxylierender Verbindung. Das erhaltene Alkoxylat kann im allgemeinen ohne vorherige Abtrennung des Katalysators eingesetzt werden.

Der erfindungsgemäße Alkoxylierungskatalysator weist eine hohe katalytische Aktivität auf und führt in relativ kurzer Reaktionszeit zu einem praktisch vollständigen Umsatz und zu hoher Ausbeute. Das Alkoxylat weist eine enge Homologenverteilung auf und ist auch farblos und häufig klar und hat damit ein gutes Aussehen. Ein weiterer Vorteil liegt darin, daß der erfindungsgemäß vorgeschlagene Katalysator auch leicht in situ gebildet werden kann.

Wenn auch die Art der Alkylenoxide und der aktive H-Atome enthaltenden Verbindungen für das erfindungsgemäße Verfahren nicht kritisch sind, sei dazu noch folgendes ausgeführt:

Als Alkylenoxide werden vorzugsweise Ethylenoxid, Propylenoxid und/oder Butylenoxid eingesetzt, wobei Ethylenoxid und/oder Propylenoxid bevorzugt sind. Besonders bevorzugt ist Ethylenoxid.

Aktive H-Atome enthaltende Verbindungen sind zum Beispiel Hydroxylgruppen enthaltende Verbindungen, Aminverbindungen und Säureverbindungen wie Fettsäuren, wobei die erstgenannten bevorzugt sind. Hydroxylgruppen enthaltende Verbindungen sind zum Beispiel Alkohole, Aminoalkohole, Perfluoralkylalkohole, Glykole, Glykolmonoether, Glycerin, Phenole, Kresole und dergleichen, wobei Alkohole bevorzugt sind. Sie können aus nativer Quelle oder aus Synthese-Prozessen stammen, primär, linear oder verzweigt, gesättigt oder ungesättigt, ein- oder mehrwertig sein, zum Beispiel Oxo-Alkohole, Guerbet-Alkohole, Ziegler-Alkohole, Fettalkohole und dergleichen. Bevorzugte Alkohole sind die primären, geradkettigen oder verzweigten $C_3$ bis $C_{24}$-Alkanole, vorzugsweise $C_6$ bis $C_{18}$-Alkanole (Fettalkohole) oder Gemische davon, zum Beispiel Gemische aus $C_{12}$ und $C_{14}$-Alkanol ($C_{12/14}$). Als Beispiele für die bevorzugten Alkohole seien genannt: Butanol, Amylalkohol, Hexanol, Nonanol, Isononylalkohol, Decanol, Undecanol, Isoundecanol, Laurylalkohol, Isotridecylalkohol, Stearylalkohol, Kokosfettalkohol und Gemische davon, ferner 2-Ethylhexanol, 2-Hexyldecanol, 2-Octyldecanol und ähnliche Guerbet-Alkohole.

Die Erfindung wird nun an Beispielen und Vergleichsbeispielen noch näher erläutert.

Die Homologenverteilung der in den Beispielen und Vergleichsbeispielen erhaltenen Alkoxylate wurde nach vorausgehender Derivatisierung mittels Kapillargaschromatographie bestimmt. Als Maß für die Homologenverteilung wird der sogenannte Q-Wert gemäß Gleichung $Q = n^* \cdot p^2$ angegeben, in der n* die durchschnittliche Adduktzahl (mittlerer Alkoxylierungsgrad) und p die Gewichtsprozente des am meisten vorkommenden Alkoxylierungshomologen bedeuten. Dieser Q-Wert ist bekanntlich insbesondere dann ein guter Maßstab, wenn es sich um Alkoxylate mit einem im wesentlichen gleichen durchschnittlichen Alkoxylierungsgrad handelt. Höhere Werte von Q zeigen eine selektivere Alkoxylierung und ein Alkoxylat mit einer engeren Homologenverteilung an.

Herstellung der Katalysatoren:

Beispiele 1A bis 10A (Vergleichsbeispiele)

1 mol Alkenylbernsteinsäureanhydrid wird mit 1 mol Alkohol, 1 mol Ethylenglykol (Beispiel 4A) oder 1 mol Triethylenglykolmonomethylether (Beispiel 3A) bei 60 bis 150 °C unter $N_2$-Atmosphäre zum entsprechenden Alkenylbernsteinsäurehalbester umgesetzt. Die Reaktionszeit beträgt abhängig vom Alkohol zwischen 3 und 25 Stunden bei einem Überdruck von 0 bis 2 bar. 1 mol so hergestellter Alkenylbernsteinsäurehalbester, 600 ml Toluol, 228 g Mineralöl und 0,55 mol Erdalkalihydroxid werden bei Raumtemperatur verrührt, und bei 100 bis 120 °C wird in circa 2 Stunden das entstehende Wasser azeotrop ausgetrieben. Nun werden 10 g Filterhilfsmittel zugesetzt, filtriert und das klare Filtrat am Rotationsverdampfer vom eingesetzten Toluol befreit. Man erhält circa 60gew.%ige Lösungen der Alkenylbernsteinsäurehalbester-Erdalkalisalze als rotbraune, klare Öle.

Beispiele 11B bis 13B (erfindungsgemäß)

Zu 100 g von einem Alkenylbernsteinsäurehalbester-Calciumsalz mit einer titrierbaren Alkalität von Alkalizahl 106 (mg KOH/g) wird das 0,4fache der zur Neutralisation der Alkalität notwendigen stöchiometrischen Menge $H_2SO_4$ (als 25gew.%ige wäßrige Lösung) zugegeben, verrührt und bei 90 °C unter Vakuum (20 mbar) vom Wasser befreit. Man erhält eine gelbe, pastöse Substanz.

In der nachstehenden Tabelle 1 sind die Beispiele 1A bis 10A und 11B bis 13B unter Bezugnahme auf die Formeln (I) und (II) zusammengefaßt:

Tabelle 1

| Beispiel | R | $R^1$ | n | M | y |
|---|---|---|---|---|---|
| 1A | iso-$C_{12}$-Alkenyl | iso-Butyl | 0 | Ca | 1 |
| 2A | iso-$C_{12}$-Alkenyl | Octyl | 0 | Ca | 1 |
| 3A | iso-$C_{12}$-Alkenyl | Methyl | 3 | Ca | 1 |
| 4A | iso-$C_{12}$-Alkenyl | H | 1 | Ca | 1 |
| 5A | iso-$C_{12}$-Alkenyl | Lauryl | 0 | Ca | 1 |
| 6A | iso-$C_9$-Alkenyl | iso-Butyl | 0 | Ca | 1 |
| 7A | n-$C_{12/14}$-Alkenyl | Methyl | 0 | Ca | 1 |
| 8A | n-$C_{18}$-Alkenyl | iso-Butyl | 0 | Ca | 1 |
| 9A | iso-$C_{12}$-Alkenyl | iso-Butyl | 0 | Ba | 1 |
| 10A | iso-$C_{12}$-Alkenyl | iso-Butyl | 0 | Sr | 1 |
| 11B | iso-$C_{12}$-Alkenyl | iso-Butyl | 0 | Ca + $H_2SO_4$ | 1 |
| 12B | iso-$C_{12}$-Alkenyl | iso-Butyl | 0 | Ca + $H_3PO_4$ | 1,8 |
| 13B | iso-$C_{12}$-Alkenyl | $C_{12/14}$-Alkyl | 0 | Ca + $H_2SO_4$ | 1 |

Alkoxylierung in Gegenwart der Katalysatoren der Beispiele 1A bis 10A und 11B und 12B:

Beispiele 1 bis 15 (davon sind die Beispiele 1 bis 10 und 12 Vergleichsbeispiele)

In einem Druckreaktor aus Glas werden 1 mol von einem Fettalkohol eingewogen (das sind zum Beispiel 194 g $C_{12/14}$-Alkanol) und durch Aufheizen und Anlegen von Vakuum beziehungsweise alternativ durch Durchleiten von Stickstoff soweit getrocknet, daß der Wassergehalt im Alkohol 0,10 Gew.-% nicht überschreitet. Dann werden 0,9 Gew.-%, bezogen auf Fettalkohol, Katalysator zugesetzt (zum Beispiel 2,9 g der 60gew.%igen Lösung von Katalysator 1A). Nach Aufheizen auf 160 °C wird mit der Zudosierung von Ethylenoxid (EO) begonnen und die gesamte vorgesehene Menge EO bei 150 bis 180 °C nach Maßgabe der Abreaktion (erkennbar an der Druckabnahme im Reaktor) aufgedrückt.

Die nach dieser Alkoxylierungs-Arbeitsweise mit den nicht erfindungsgemäßen Katalysatoren 1A bis 10A (Beispiele 1 bis 10 und 12) und mit den erfindungsgemäßen Katalysatoren 11B und 12B (Beispiele 11 und 13 bis 15) erhaltenen Ergebnisse sind in der nachfolgenden Tabelle 2 zusammengefaßt, die auch das nachfolgende erfindungsgemäße Beispiel 16 enthält.

Beispiel 16 (in-situ-Bildung des Katalysators)

In einem Druckreaktor aus Glas werden 194 g $C_{12/14}$-Alkanol (1 mol) eingewogen und 1,95 g iso-$C_{12}$-Alkenylbernsteinsäureanhydrid (7,34 mmol) zugesetzt. Nach kurzem Verrühren werden 0,28 g 97gew.%ige $Ca(OH)_2$ (3,67 mmol) zudosiert und 1 Stunde bei 130 °C nachgerührt. Dann werden bei 90 °C 1,96 g 11gew.%ige $H_2SO_4$ zugegeben (2,20 mmol), das entspricht einer circa 60%igen Neutralisation der Erdalkali-Basizität. Nun wird etwa 2 Stunden bei 130 °C bei vermindertem Druck (Restdruck circa 20 mbar) gerührt, um das Wasser zu entfernen. Anschließend wird bei einer Temperatur von 150 bis 170 °C die Umsetzung mit dem Epoxid durchgeführt.

Tabelle 2

| Beispiel | Katalysator | Katalysatormenge (Gew.-%) | Startalkohol | mol EO/mol Alkohol | Q-Index |
|---|---|---|---|---|---|
| 1 | 1A | 0,9 | n-$C_{12/14}$ | 4 | 1100 |
| 2 | 2A | 0,9 | n-$C_{12/14}$ | 4 | 1000 |
| 3 | 3A | 0,9 | n-$C_{12/14}$ | 4 | 1020 |
| 4 | 4A | 0,9 | n-$C_{12/14}$ | 4 | 1050 |
| 5 | 5A | 0,9 | n-$C_{12/14}$ | 4 | 1000 |
| 6 | 6A | 0,9 | n-$C_{12/14}$ | 4 | 1000 |
| 7 | 7A | 0,9 | n-$C_{12/14}$ | 4 | 1030 |
| 8 | 8A | 0,9 | n-$C_{12/14}$ | 4 | 1030 |
| 9 | 9A | 0,9 | n-$C_{12/14}$ | 4 | 1000 |
| 10 | 10A | 0,9 | n-$C_{12/14}$ | 4 | 1000 |
| 11 | 12B | 0,9 | n-$C_{12/14}$ | 4 | 1850 |
| 12 | 1A | 0,9 | n-$C_{16/18"}$ | 8 | 1350 |
| 13 | 11B | 0,9 | n-$C_{12/14}$ | 4 | 1840 |
| 14 | 11B | 0,9 | n-$C_{12/14}$ | 6 | 2400 |
| 15 | 11B | 0,9 | n-$C_{12/14}$ | 11 | 3500 |
| 16 | in-situ-Bildung | 1,0 | n-$C_{12/14}$ | 4 | 1770 |

Die Alkoxylate der Beispiele 1 bis 10 und 12 sind klar und jene der Beispiele 11 und 13 bis 16 sind leicht trüb.

Weitere Vergleichsbeispiele 1 und 2

Die Ethoxylierungen der Beispiele 1 und 14 wurden wiederholt (das heißt Ethoxylierung mit 4 beziehungsweise 6 mol EO pro mol Startalkohol), wobei als Katalysator ein Calciumsalz des unsubstituierten Bernsteinsäure-diethylenglykolmonoethylether-Halbesters eingesetzt wurde, das ist der Katalysator Nr. 2 der eingangs genannten EP-A 0 337 239.

Diese Vergleichsbeispiele sind in der folgenden Tabelle 3 zusammengefaßt:

Tabelle 3

| Vergleichsbeispiel | Katalysatormenge (Gew.-%) | Startalkohol | mol EO/mol Alkohol | Q-Index |
|---|---|---|---|---|
| 1 | 0,9 | n-$C_{12/14}$ | 4 | 1000 |
| 2 | 0,9 | n-$C_{12/14}$ | 6 | 1300 |

Die Alkoxylate der Vergleichsbeispiele 1 und 2 sind stark trüb.

**Patentansprüche**

1. Verfahren zur Herstellung von Alkoxylaten durch Alkoxylierung von mindestens ein aktives H-Atom enthaltenden Verbindungen in Gegenwart eines Salzes von einem Bernsteinsäuremonoester als Katalysator, dadurch gekennzeichnet, daß man in Gegenwart von mindestens einem Erdalkalimetallsalz der Alkyl- oder Alkenylbernsteinsäuremonoester der nachstehenden Formeln (I) und (II) alkoxyliert

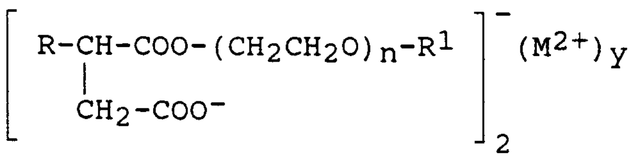

(I)

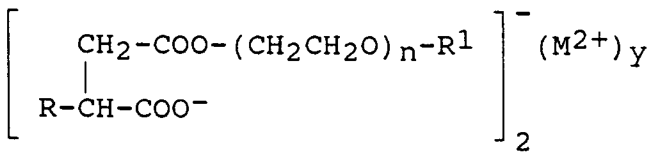

(II)

worin bedeuten

R — $C_8$ bis $C_{30}$-Alkyl oder $C_8$ bis $C_{30}$-Alkenyl,
n — eine Zahl von 0 bis 6,
$R^1$ — $C_1$ bis $C_{18}$-Alkyl oder $C_3$ bis $C_{18}$-Alkenyl oder auch Wasserstoff, wenn n 1 oder > 1 ist,
M — Ba, Ca oder Sr und
y — eine Zahl von 0,9 bis 1,8,

wobei 10 bis 70 % der titrierbaren Alkalitat des Bernsteinsäuremonoestersalzes der Formeln (I) und (II) mit einer anorganischen Säure, die mit den Kationen Ba, Ca und Sr in Wasser schwerlösliche Salze bildet, neutralisiert worden sind.

**2.** Verfahren nach Anspruch 1, dadurch gekennzeichnet, daß R ein C9 bis C20-Alkyl oder ein $C_9$ bis $C_{20}$-Alkenyl ist, n eine Zahl von 0 bis 3, $R^1$ ein $C_1$ bis $C_{12}$-Alkyl oder ein $C_3$ bis $C_{12}$-Alkenyl oder das Wasserstoffatom ist, wenn n 1 oder > 1 ist, und y eine Zahl von 1 bis 1,3 ist.

**3.** Verfahren nach Anspruch 1 oder 2, dadurch gekennzeichnet, daß man in Gegenwart von mindestens einem Bernsteinsäuremonoestersalz der Formeln (I) und (II) alkoxyliert, das durch Umsetzung der Bernsteinsäuremonoesterverbindung mit einem Oxid, Carbonat oder Hydroxid des Bariums, Calciums oder Strontiums in einer Menge von 0,9 bis 1,8 mol pro 2 mol Bernsteinsäuremonoester erhalten worden ist.

**4.** Verfahren nach einem oder mehreren der Ansprüche 1 bis 3, dadurch gekennzeichnet, daß als anorganische Säuren schwefelige Säure, Schwefelsäure, phosphorige Säure oder Phosphorsäure eingesetzt wird.

**5.** Verfahren nach einem oder mehreren der Ansprüche 1 bis 4, dadurch gekennzeichnet, daß der Alkoxylierungskatalysator in einer Menge von 0,1 bis 5 Gew.-% vorliegt, bezogen auf die Menge an zu alkoxylierender Verbindung.

**6.** Verfahren nach einem oder mehreren der Ansprüche 1 bis 5, dadurch gekennzeichnet, daß Hydroxylgruppen enthaltende Verbindungen mit Ethylenoxid oder Propylenoxid oder mit einer Mischung der beiden alkoxyliert werden.

**7.** Verfahren nach einem oder mehreren der Ansprüche 1 bis 5, dadurch gekennzeichnet, daß Fettalkohole mit Ethylenoxid oder Propylenoxid oder einer Mischung der beiden alkoxyliert werden.

**8.** Verfahren nach einem oder mehreren der Ansprüche 1 bis 7, dadurch gekennzeichnet, daß das Alkylenoxid in einer Menge von 2 bis 15 mol pro mol zu alkoxylierender Verbindung eingesetzt wird.

## Claims

**1.** A process for the preparation of alkoxylates by alkoxylating compounds containing at least one active hydrogen atom in the presence of a salt of a succinic monoester as catalyst, which comprises carrying out the alkoxylation in the presence of at least one alkaline earth metal salt of an alkyl- or alkenylsuccinic monoester of the formulae (I) and (II) below

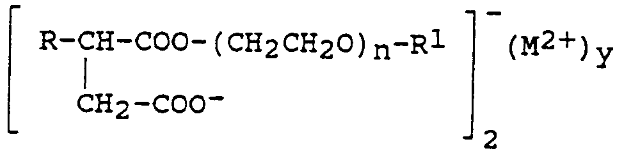

(I)

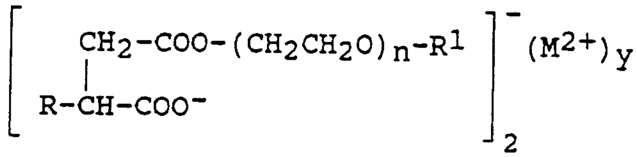

(II)

in which

R is $C_8$ to $C_{30}$ alkyl or $C_8$ to $C_{30}$ alkenyl,
n is a number from 0 to 6,
$R^1$ is $C_1$ to $C_{18}$ alkyl or $C_3$ to $C_{18}$ alkenyl or hydrogen, if n is 1 or > 1,
M is Ba, Ca or Sr, and
y is a number from 0.9 to 1.8,

wherein from 10 to 70 % of the titratable alkalinity of the succinic monoester salt of the formulae (I) and (II) have been neutralized using an inorganic acid which forms salts of low solubility in water with the cations Ba, Ca and Sr.

**2.** The process as claimed in claim 1, wherein R is a $C_9$ to $C_{20}$ alkyl or a $C_9$ to $C_{20}$ alkenyl, n is a number from 0 to 3, $R^1$ is a $C_1$ to $C_{12}$ alkyl or a $C_3$ to $C_{12}$ alkenyl, or a hydrogen atom if n is 1 or > 1, and y is a number from 1 to 1.3.

**3.** The process as claimed in claim 1 or 2, wherein alkoxylation is carried out in the presence of at least one succinic monoester salt of the formulae (I) and (II) which has been obtained by reacting the succinic monoester compound with an oxide, carbonate or hydroxide of barium, calcium or strontium in a quantity of from 0.9 to 1.8 mol per 2 mol of succinic monoester.

**4.** The process as claimed in one ore more of claims 1 to 3, wherein the inorganic acid employed is sulfurous acid, sulfuric acid, phosphorous acid or phosphoric acid.

**5.** The process as claimed in one or more of claims 1 to 4, wherein the alkoxylation catalyst is present in a quantity of from 0.1 to 5 % by weight based on the quantity of compound to be alkoxylated.

**6.** The process as claimed in one or more of claims 1 to 5, wherein compounds containing hydroxyl groups are alkoxylated using ethylene oxide or propylene oxide or a mixture of the two.

**7.** The process as claimed in one or more of claims 1 to 5, wherein a fatty alcohol is alkoxylated with ethylene oxide or propylene oxide or a mixture of the two.

**8.** The process as claimed in one or more of claims 1 to 7, wherein the alkylene oxide is employed in a quantity of from 2 to 15 mol per mole of compound to be alkoxylated.

## Revendications

**1.** Procédé pour préparer des produits d'alcoxylation par alcoxylation de composés contenant au moins un atome d'hydrogène actif, en présence d'un sel d'un monoester de l'acide succinique en tant que catalyseur, caractérisé en ce qu'on procède à l'alcoxylation en présence d'au moins un sel d'un métal alcalino-terreux de monoesters d'un acide alkyl- ou alcénylsuccinique ayant les formules (I) et (II) ci-après

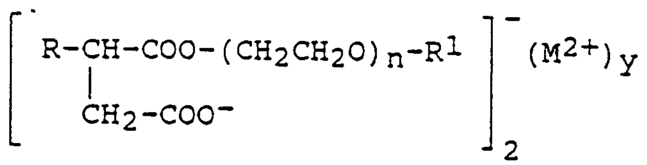

(I)

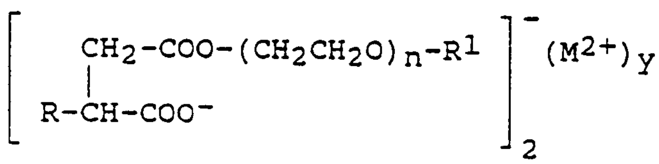

(II)

dans lesquelles :

R est un groupe alkyle en $C_8$ à $C_{30}$ ou alcényle en $C_8$ à $C_{30}$,
n est un nombre de 0 à 6,
$R^1$ est un groupe alkyle en $C_1$ à $C_{18}$ ou alcényle en $C_3$ à $C_{18}$, ou encore un atome d'hydrogène quand n vaut 1 ou est > 1,
M est Ba, Ca ou Sr, et
y est un nombre de 0,9 à 1,8

où 10 à 70 % de l'alcalinité titrable du sel d'un monoester de l'acide succinique, ayant les formules (I) et (II), ont été neutralisés avec un acide minéral qui forme avec les cations Ba, Ca et Sr des sels difficile-ment solubles dans l'eau.

**2.** Procédé selon la revendication 1, caractérisé en ce que R est un groupe alkyle en $C_9$ à $C_{20}$ ou alcényle en $C_9$ à $C_{20}$, n est un nombre de 0 à 3, $R^1$ est un groupe alkyle en $C_1$ à $C_{12}$ ou alcényle en $C_3$ à $C_{12}$ ou l'atome d'hydrogène, quand n vaut 1 ou est > 1, et y est un nombre de 1 à 1,3.

**3.** Procédé selon la revendication 1 ou 2, caractérisé en ce qu'on procède à l'alcoxylation en présence d'au moins un sel d'un monoester de l'acide succinique ayant les formules (I) et (II), que l'on a obtenu par réaction du composé monoester de l'acide succinique avec un oxyde, un carbonate ou un hydroxyde de baryum, de calcium ou de strontium en une quantité de 0,9 à 1,8 moles pour 2 moles du monoester de l'acide succinique.

**4.** Procédé selon l'une ou plusieurs des revendications 1 à 3, caractérisé en ce qu'on utilise en tant qu'acides minéraux l'acide sulfureux, l'acide sulfurique, l'acide phosphoreux ou l'acide phosphorique.

**5.** Procédé selon l'une ou plusieurs des revendications 1 à 4, caractérisé en ce que le catalyseur d'alcoxylation est présent en une quantité de 0,1 à 5 % en poids par rapport à la quantité du composé à alcoxyler.

**6.** Procédé selon l'une ou plusieurs des revendications 1 à 5, caractérisé en ce que les composés contenant des groupes hydroxyle sont alcoxylés avec de l'oxyde d'éthylène ou de l'oxyde de propylène ou un mélange des deux.

**7.** Procédé selon l'une ou plusieurs des revendications 1 à 5, caractérisé en ce qu'on alcoxyle des alcools gras avec de l' oxyde d'éthylène ou de l'oxyde de propylène ou un mélange des deux.

**8.** Procédé selon l'une ou plusieurs des revendications 1 à 7, caractérisé en ce que l'oxyde d'alkylène est utilisé en une quantité de 2 à 15 moles par mole du composé à alcoxyler.